Europäisches Patentamt

European Patent Office

Office européen des brevets

(19)

(11) Publication number: **0 083 095**

**A2**

(12)

# EUROPEAN PATENT APPLICATION

(21) Application number: **82111986.4**

(22) Date of filing: **23.12.82**

(51) Int. Cl.³: **A 61 K 7/09**

(30) Priority: **24.12.81 US 334307**

(43) Date of publication of application:
**06.07.83 Bulletin 83/27**

(84) Designated Contracting States:
**AT BE DE FR GB IT**

(71) Applicant: **Helene Curtis Industries, Inc.**
**4401 West North Avenue**
**Chicago, Ill. 60639(US)**

(72) Inventor: **Hsiung, Du Yung**
**130 Illinois Street**
**Park Forest Illinois 60466(US)**

(72) Inventor: **Davis, Chester A.**
**3240 East Avenue**
**Berwyn Illinois 60403(US)**

(74) Representative: **Groening, Hans Wilhelm, Dipl.-Ing.**
**Siebertstrasse 4 Postfach 860 340**
**D-8000 München 86(DE)**

(54) **Method of reductively waving or straightening hair.**

(57) A method of reductively waving or straightening hair is disclosed in which a first composition containing a reducing agent capable of breaking hair disulfide is applied to the hair and maintained in contact with the hair to form partially reduced hair. The first composition is rinsed from the hair, and the temperature of the hair is raised and maintained at from about 35° C. to about 100° C., followed by neutralization to rebuild the hair disulfide bonds.

EP 0 083 095 A2

# METHOD OF REDUCTIVELY WAVING OR STRAIGHTENING HAIR
## Description
### Technical Field

The present invention relates to waving or straightening hair, and particularly to a method in which hair disulfide bonds are cleaved by a reducing agent and allowed to process at an elevated temperature in the absence of the reducing agent.

### Background Art

Several methods are known in the art for reductively waving or straightening hair. In following these methods, a reducing agent capable of breaking hair keratin disulfide bonds is applied to the hair and maintained in contact with the hair until a sufficient number of hair disulfide bonds are broken so that after the configuration of the hair is changed, and the hair then neutralized, a wave (curl) or straightening will be noted in the hair. The composition containing the reducing agent is typically called a waving lotion, and the waving lotion is usually maintained in contact with the hair to be waved for up to about 30 minutes at ambient, room temperature, or for shorter periods of times if the hair and waving lotion admixture are heated. After a sufficient number of disulfide bonds in the hair are broken by the reductive process and the configuration of the hair is changed, the hair is then rinsed of substantially all of the waving lotion, and the broken hair keratin disulfide bonds in the altered hair configuration are rebuilt by application of a neutralizing solution to maintain the hair in the new configuration.

Hair to be waved or straightened is usually placed under a tensile force from an external source, such as by winding on rollers or by combing, prior to the disulfide bonds being rebuilt to assist in accomplishing the configurational change. Rollers of a relatively small diameter are usually used to impart a wave, or curl, while rollers of a relatively large diameter and/or combing are usually used for straightening.

One of the problems associated with reductive hair waving or straightening is overwaving, also known as overprocessing. Hair which is overprocessed tends to be more brittle and break more easily than is hair which is waved to the proper degree. Overwaving, or exposure of the hair to a reducing step of excessive severity, generally stems from a desire on the part of the user to ensure a proper degree of waving, and in so doing, utilizing a waving lotion having an amount of reducing agent which is excessive for the hair to be waved or straightened, leaving the waving lotion with a proper amount of reducing agent on the hair for too long a period of time, or the like. Overprocessing has been controlled to some degree by the use of compositions which contain water soluble disulfide compounds. However, not all waving processes utilize such compositons.

Another problem which can arise in reductive waving or straightening is that of irritation to the user's skin caused by contact of the scalp or neck area with the waving lotion. While skin irritation is only infrequently observed, it is a potential problem which must be dealt with by manufacturers of reductive waving and straightening products. One way that manufacturers have dealt with this problem is to treat the hair with waving lotion, and then allow the hair to process at about body temperature in the substantial absence of waving lotion. However, waves so produced tend to require a relatively long processing time.

-3-

Yet another problem faced by the industry is that the waving or straightening effects which are induced tend to be lost or diminished after the hair is shampooed several times. Additionally, and aside from the effect of shampoos, waved or straightened hair tends to revert with time to approach its original configuration.

It would therefore be beneficial if reductive waving or straightening could be carried out with a minimized risk of overprocessing. It would also be advantageous if the already low risk of skin irritation on the part of the users of reductive waving and straightening products could be minimized still further. It would be additionally beneficial if the effects of a waving or straightening treatment were longer lasting than is usually obtained.

Brief Description of the Invention

A new method for reductively waving or straightening hair is disclosed. According to this method, a first composition containing a reducing agent capable of reductively breaking the disulfide bonds in hair keratin is applied to contact hair fibers. This first composition is maintained in contact with the hair fibers to form partially reduced hair, i.e., for a period of time sufficient to break enough hair disulfide bonds to produce a change in configuration if the hair is wound on rollers and then neutralized. Preferred compositions can contain from about 0.5 to 30 weight percent of reducing agent and such compositions are preferably maintained in contact with the hair for a period from about two to about thirty minutes. The first composition is then substantially completely rinsed from the partially reduced hair.

-4-

Thereafter, the temperature of the rinsed, partially reduced hair is raised to from about 35° C. to about 100° C. and the hair is maintained at that raised temperature for a period of about one to sixty minutes. A second, neutralizing, composition is then applied to the heated hair to rebuild the broken hair disulfide bonds and form waved or straightened hair.

One of the salient features of the present invention is that the temperature of the hair is raised after the waving lotion; i.e. the first composition containing a reducing agent capable of breaking hair keratin disulfide bonds, is rinsed from the partially reduced hair. This heating after rinsing is not employed in any prior process for waving or straightening hair which has been previously practiced, and leads to several surprising and unanticipated benefits and advantages.

One of these benefits is that after waving the hair to a particular amount of curl, the condition of the hair treated by the method of this invention is better than is the condition of similar hair waved to about the same amount of curl using a previously known method of waving.

Another benefit of the present invention is that while maintaining hair condition at an approximately constant level, a deeper wave and/or more wave can be imparted than by using the conventional method.

Another advantage of the present invention is that the curls imparted are longer lasting than are those imparted by the usual process.

It is emphasized that the benefits and advantages of this invention to be waved (curled) or straightened hair, are the result of a method which is carried out in the substantial absence of the

-5-

usually present reactants, rather than in the presence of a larger amount of those reactants, and therefore represent truly unexpected results.

Yet another advantage of the present invention is that contact of the user's skin with waving lotion can be substantially reduced, thereby lessening the potential for irritation.

Still other benefits and advantages of the present invention will be clear to those skilled in the art from the detailed description, examples, and claims which follow.

Detailed Description of the Invention

The present invention relates to a method for waving or straightening hair in which reducing agents are used to cleave disulfide bonds within the hair. As used herein, the word "reductive," and its various grammatical forms, used in conjunction with the words "waving" and/or "straightening" is meant to differentiate the method of this invention from those hair treating processes which are carried on at high pH values, e.g. about pH 11 to about pH 14, and which occur primarily by the transformation of hair keratin disulfide bonds into lanthionine bonds. Typical agents used in "reductive hair waving or straightening" are discussed hereinafter, as is the mechanism by which is believed that those agents act upon the hair during the method of this invention.

The method of this invention relates to both waving; i.e., adding curl to hair, and also to straightening; i.e., lessening of the amount of curl in the hair. Since both waving and straightening can be carried out using the same method steps, both waving and straightening will be hereinafter be referred to as "waving", and any special differences between either adding curl or lessening the amount of curl in hair will be particularly pointed out, as required.

In carrying out the method of the present invention, a first composition (waving lotion) containing a reducing agent capable of reductively breaking the disulfide bonds of hair keratin is applied to the hair to contact the hair fibers. This solution is typically an aqueous solution and the reducing agent therein is selected from the well known group of reducing agents.

The particularly preferred reducing agents are water-soluble thiol-containing agents such as mercaptocarboxylic acids, their alkali metal and ammonium salts, their esters, and mixtures thereof. Examples of these reducing agents include thioglycolic acid, thiolactic acid, 3-mercaptopropionic acid and the like. Suitable alkali metal salts of these acids include sodium or potassium salts. Ammonium salts can also be used, and include salts prepared from these acids and ammonia, mono-, di- or triethanolamine and mono-, di- or triiso-propanolamine. Particularly preferred mercapto carboxylic acid esters include glyceryl thioglycolate.

Bisulfite ion, present in the waving lotion as a water-soluble alkali metal or ammonium bisulfite, is also a particularly preferred reducing agent herein. The cleavage of hair disulfide bonds using the bisulfite ion in waving processes is sometimes referred to as "sulfitolysis." Since bisulfite ion is a well known reducing agent, and since its action upon the hair includes production of hair keratin thiol groups which remain in the hair during the change in the configuration of the hair, bisulfite ion is included herein as a reducing agent.

The reducing agents are preferably present in the waving lotion from 0.5 to about 30 percent by weight of the waving lotion. When the particularly preferred thiol-containing acids or acid salts are utilized, they are preferably present at about 1 to about 20 weight percent of the waving lotion, measured as the free acid. Thio esters, such as glyceryl thioglycolate, are particularly preferred to be utilized at about 3 to about 30 weight percent of the waving lotion. Alkali metal or ammonium bisulfites are preferably present in the waving lotion at from about 5 to about 15 weight percent, and more preferably from about 10 to about 13 weight percent.

The pH values of waving lotions useful herein are known in the art for use with particular reductive waving agents. For example, compositions, which include mercaptocarboxylic acid salts, such as ammonium thioglycolate, typically have a pH value of from about 7.5 to about 9.5, while compositions including mercaptocarboxylic acid esters, such as glyceryl thioglycolate typically have pH values of about 6 to about 7.5. Waving lotions which contain bisulfite ion, such as those containing ammonium bisulfite, are typically used at a pH value of about 6.5 to about 8.

The waving lotion, or first composition, is maintained in contact with the hair for period of time sufficient to form partially reduced hair; i.e., to break enough of the hair keratin disulfide bonds to produce a change in configuration if that hair is wound on rollers and then neutralized. The amount of time required to produce a permanent wave using the above described compositions and agents is well known in the art.

-8-

When using the particularly preferred thiol-containing agents, the first composition is preferably maintained in contact with hair for a period of from about two to about thirty minutes, and more preferably for about two to about twenty minutes. When a waving lotion containing bisulfite ion is used, the lotion is typically maintained in contact with the hair for from about ten to about thirty minutes. The hair can be heated while in contact with a reducing agent, such as bisulfite ion, during the production of partially reduced hair. However, this heating step is not to be confused with the hair heating step which is carried out after the partially reduced hair has been formed and the reducing agent is substantially absent from the hair, as is further discussed hereinafter.

Examining the duration of treatment with the first composition from the viewpoint of the amount of hair disulfide bonds broken, the first composition is maintained in contact with the hair until at least about ten percent of the hair disulfide bonds are broken. More preferably, at least about fifteen percent of the hair keratin disulfide bonds and no more than about sixty percent of said bonds are broken by the contact maintained between the hair and first composition. Methods for determining the amount of hair disulfide cleavage are well known in the art.

It is noted that the concentration of reducing agent within the waving lotion, the pH value of the lotion, the time the waving lotion is maintained in contact with the hair and the temperature at which the waving lotion is maintained in contact with the hair are all interrelated and are a function of the condition of the hair to be waved, and the degree of waving desired. For example, virgin hair, which has had no chemical treatment other than shampooing, typically requires more stringent conditions to effect a wave than does more porous hair which has received a previous chemical treatment, such as bleached hair. These factors are usually taken into consideration by waving product manufacturers as is evidenced by commercial products having differing strengths and processing instructions for use on hair of differing histories.

After the partially reduced hair is formed, the first composition is rinsed substantially completely from the partially reduced hair. The amount of rinsing needed at this step is that amount known to skilled practitioners in the art, and is the amount used when the waving lotion is rinsed from the hair in a conventional waving process prior to the neutralization step. In usual practice, about three minutes of rinsing in a sink using running warm tap water is sufficient to rinse the hair substantially free of the first, reducing agent-containing, composition. As used herein, the term "rinse" in any of its grammatical forms includes treatment with a shampoo.

After the waving lotion has been substantially rinsed from the hair, the rinsed, damp, partially reduced hair is raised in temperature to from about 35° C. to about 100° C., and preferably from about 40° C. to about 55° C. Heating of the hair to about 100° C. is contemplated particularly in the case of wigs made from human hair. The temperatures to which the hair are raised can be measured by insertion of a thermometer or thermocouple into the heated hair mass.

Methods for raising the hair temperature typically include covering the user's hair with a plastic cap and then heating the hair with a hair

-10-

dryer, heat lamp, or with heated clamps as described in U.S. Patents No. 3,889,097, and No. 3,885,577. Another contemplated method for heating the hair utilizes preheated rollers or curling rods upon which the hair is wound so that the heat of the rollers can be imparted to the hair. Of course, any other convenient method for heating the hair can be employed.

The hair temperature is preferably raised and maintained at an elevated temperature for a total time period of from about one to about sixty minutes, and more preferably for a time period of from about two to about thirty minutes.

It is noted again that raising the temperature of the partially reduced hair after the waving lotion is substantially completely rinsed from the hair, and maintaining the hair at the raised temperature to impart a new configuration to the hair has not heretofore been practiced in the waving arts to the knowledge of the present inventors. Rather, the hair is usually heated while in contact with the applied waving lotion and then rinsed. It is believed that the new method of carrying out the steps of the waving process disclosed herein, and particularly the application of heat in the substantial absence of waving lotion, is chiefly responsible for the unusual results which are obtained by this process. A proposed mechanism which is believed to underlie the improved results obtained in the practice of this invention is discussed hereinafter. However, it is not intended to be bound by any particular theory, or mechanism.

The final step required in the process of the present invention is neutralization of the partially reduced, heated hair by the application of

-11-

a second composition to rebuild the broken hair keratin disulfide bonds to form the waved hair. Several means of rebuilding hair keratin disulfide bonds which have been cleaved by reducing agents are known in the art. For example, when thiol-containing agents (acids, acid salts or esters) are utilized to break the disulfide bonds, oxidizing agents, such as hydrogren peroxide, perborate or bromate salts, such as sodium perborate and sodium bromate, are well known to be capable of rebuilding broken hair keratin disulfide bonds.

When bisulfite ion is utilized as a reductive, disulfide bond breaking agent, it is well known that the hair can be neutralized and the hair keratin disulfide bonds remade by treating the partially reduced hair with an aqueous composition having a pH value of from about 8.5 to about 10.5. Other agents such as water soluble disulfide salts can also be utilized in the reformation of hair disulfide bonds from partially reduced hair prepared using bisulfite ion, as can oxidizing agents, such as hydrogen peroxide.

The concentration of agents within the second composition and the pH value of that composition are well known to those skilled in the hair waving arts and need not be discussed fully herein. However, when the preferred, thiol-containing agents are utilized in reductively breaking the hair keratin disulfide bonds, an aqueous hydrogen peroxide solution is preferred for rebuilding the hair keratin disulfide bonds. The hydrogen peroxide is preferably used at a concentration of about 1 to about 3 weight percent of the second composition and at a pH value from about 3 to about 6.

-12-

Hair treated in accordance with this invention is subjected to a tensile force during the reorientation, or "creep" step (defined hereinafter). The tensile force can be applied internally by the weight of damp hair, or externally to the hair. Utilization of an externally applied tensile force to the hair is preferred. Examples of externally applied tensile forces include the force provided by wrapping the hair on rollers or curling rods (hereinafter rollers) as is usually carried out in the waving and straightening arts, and the force applied by combing the damp hair, as is carried out in straightening. Wrapping of the hair on rollers is particularly preferred.

When used in this invention, the application of a tensile force to the hair can be carried out at any time prior to the heating step during which the temperature of the hair is raised in the substantial absence of waving lotion. It should be understood that if the tensile force is applied prior to the formation of partially reduced hair, the force on the hair is maintained, at least in part, subsequent to the formation of the partially reduced hair so that the tensile force can assist in reorienting the hair. For example, (a) the hair to be waved can be wrapped on rollers prior to one or more applications of the first, reducing agent-containing composition, or (b) the first composition can be applied and the hair wrapped thereafter, followed by maintenance of first composition-hair contact and rinsing.

In a third procedure, the partially reduced hair is prepared, the waving lotion rinsed substantially from the hair and the tensile force thereafter applied. Thus, the first composition, waving lotion, can be applied and maintained in contact with the hair while the hair is in an unwound configuration, e.g., piled on the head or free hanging, and then rinsed and the tensile force applied. Alternatively, the hair can be wound on rollers of a first diameter and partially reduced hair formed. Thereafter, the partially reduced hair is rinsed of substantially all of the reducing agent, taken off of the first rollers and then wrapped on rollers of a second (larger or smaller) diameter prior to raising the temperature of the hair.

Contact between the waving lotion and the user's skin is particularly minimized in the latter, third procedure; i.e., wrapping after rinsing, because the hair-wrapped rollers which touch the user's scalp contain substantially no waving lotion. Consequently, the already small possibility for skin irritation which might otherwise be present is further minimized. This small possibility of skin irritation is also minimized in the other wrapping procedures useful herein because the heating step, which normally occurs in the presence of waving lotion and which would also tend to enhance irritation, is carried out in the absence of waving lotion in the practice of this invention.

The mechanism by which the method of the present invention produces its advantageous and beneficial results is not known with certainty. While not desiring to be bound by any hypothesis or belief, applicants' explanation for the unusual results of their method is disclosed hereinafter. However, before explaining how applicants' process is believed to operate, a short discussion of reductive hair waving phenomena, generally, is in order. This discussion will deal only with the impartation of

-14-

curl-to the hair, but it should be understood that the phenomena discussed are equally applicable to hair straightening treatments.

When the reductive disulfide bond breaking agent is applied to the hair and absorbed, it reacts to break the hair keratin disulfide bonds. The amount of hair keratin disulfide bond breakage required to produce a wave can be as high as about eighty percent of the disulfide bonds present in the hair. It is known that even when a large excess of bond breaking agent is used, some of the hair keratin disufide bonds are not cleaved, possibly because of steric restraints. Thus, during waving, the hair contains both intact disulfide bonds and broken disulfide bonds (e.g. keratin thiol groups) as a result of treatment with a reductive disulfide bond breaking agent, such as bisulfite ion or a thiol-containing compound like thioglycolic acid or glyceryl thioglycolate.

The next principal step in a conventional waving process involves a reorientation of the hair to form a new configuration.

When an externally applied tensile force is placed on a hair fiber as by winding or wrapping the hair upon a roller, or by combing, the hair tends to reorient itself more rapidly and to a greater extent to relieve the applied tensile force on the fiber then when subjected to the tensile force resulting only from the weight of the damp hair. Heating the hair at this stage, in the presence of reducing agent, accelerates the reorientation process still further. This reorientation to relieve the applied force is allowed to continue to lessen the difference between the forces which occur naturally within the hair fiber and any external forces.

The presence of the waving lotion in the hair during a heated, reorientation step also continues breaking the disulfide bonds originally present. This continued disulfide bond breaking produces a relatively high content of keratin thiol in the hair and is believed, in part, to lead to overprocessing.

In a conventional process, the waving lotion is then rinsed from the hair. The neutralizing, second, composition is thereafter applied to the hair to rebuild substantially all of the keratin thiol groups remaining in the hair into new keratin disulfide groups.

It is emphasized that in the above described usual procedure, the waving lotion, and consequently the disulfide bond breaking agent, is in contact with the hair during the reorientation stage, and is only rinsed from the hair after that reorientation step has been finished.

Contrarily, as already noted, rinsing occurs in the instant process prior to heating, although the principal chemical reactions, involving the disulfide bond breaking, reorientation and disulfide bond rebuilding, are thought to be basically the same as those discussed above. However, it is believed that many of the unexpected benefits and advantages of the instant process stem from this novel difference in the sequence of steps. Reorientation of the keratin disulfide and thiol groups under heat and in the substantial absence of the reductive, disulfide bond breaking agent is defined herein as the "creep" step.

One of the principal features of this difference in step sequence of this invention is that the reductive disulfide bond breaking agent is not present during the creep step. Thus, the bond

-16-

breaking agent is not present during the creep step reorientation.

It is believed that part of the effectiveness of the process of the instant invention flows from the substantial absence of waving lotion during the heated, reorientation, or creep step. Thus, when partial reduction of the hair is allowed to proceed to about the same level as that achieved in the past, a better wave is obtained, with a similar resulting hair condition. On the other hand, a given amount of wave can be obtained with better hair condition from hair that is reduced to a lower level of reduction in accordance with this invention than the level of reduction normally needed to produce that given amount of wave.

It is noted that the method of the present invention can differ in result from waving processes in which the reorientation step is allowed to proceed at about ambient temperature in the presence of reducing agent. Thus, a deeper, longer lasting wave can be produced from the instant process than from prior processes in which there is no heating of the hair fibers during the reorientation step. This effect is illustrated in Example 1, hereinafter.

While heating, and therefore increasing the rate of reorientation, during the reorientation step may play some role in the improved waving observed with the method of this invention, a mere increase in the reaction rate is not thought to account for the observed differences. For instance, Example 5 discusses a comparison of processes in which one set of tresses was maintained at room temperature for six minutes after processing, followed by neutralization, while another set of tresses was heated for six minutes after the same processing time and then neutralized. The set of tresses maintained at room temperature had no curl while those tresses treated by the method of this invention had good, deep waves. If reaction rate were the only difference between the two processes, one would have expected the tresses maintained at room temperature to have more curl than was observed. It is therefore thought that some difference other than reaction rate must also play a significant role in producing the observed effect on waving.

Another phenomenon which may participate in causing the effects observed in the method of this invention is the heat assisted formation of new bonds in the damp hair. Such a reaction may be similar to that observed in wool in which a configurational set that is relatively stable to humidity can be obtained by treatment of the fibers with steam, boiling water and the like.

It may also be possible that heating in the absence of waving lotion supplies sufficient energy to cause a reorientation of secondary bonds, such as ionic or hydrogen bonds between and among the hair keratin polymer chains, and thereby contributes to the depth of the wave produced. New, and relatively stable secondary bonds may form within the hair fiber during the method of this invention because the amount of keratin-constraining disulfide bond cross-links are lessened by the partial reduction of the disulfide bonds and because the creep step of this invention occurs in the substantial absence of possibly interfering ionic ingredients from the waving lotion. This type of mechanism might be analogous to the heat and moisture-induced, relatively permanent, configurational changes noted in polyamide fibers which contain no covalent

-18-

cross-links, but which do contain hydrogen bonds between individual polymer molecules. It is also possible that the creep step provides deep waves by causing plastic flow of the less cross-linked keratin polymer chains in the partially reduced hair.

Yet another possible explanation for the observed deep, long lasting waves of this invention which are produced without substantial overprocessing relates to the neutralization step, and the effects which flow from neutralizing warm, only slightly damp hair. In the preferred embodiments of the present invention, the hair on rollers is neutralized after the creep step with no intermediate rinsing step therebetween. The partially reduced hair to be neutralized is therefore relatively dry, or has a lower moisture content than partially reduced hair that had been rinsed just prior to neutralization. In addition, the hair would possibly be at a higher temperature than hair which had been rinsed following prior procedures, as rinsing is typically carried out at a lower temperature than the preferred temperatures to which the hair is raised during the creep step, e.g., about 40°C. to about 55°C.

It is believed that the relatively dry, warm, partially reduced hair produced in accordance with this invention may produce relatively faster and possibly more complete neutralization than hair with a higher moisture content. In addition, it is thought that the neutralizer penetrates to the inner fibers on the rollers (closest to the roller itself) more readily because of the relative warmth and dryness of the hair.

This invention is further illustrated by the examples which follow.

-19-

## Best Modes For Carrying Out The Invention Comparative Tress Ammonium

Example 1: Thioglycolate Waving

Standard, virgin (unchemically treated) brown hair tresses (DeMeo Brothers, New York) were waved using a conventional, commercially available waving lotion containing ammonium thioglycolate (7.5 percent as thioglycolic acid) and having a pH value of 9.4. The tresses were neutralized using the hydrogen peroxide neutralizer solution supplied with the product.

More specifically, the tresses were first shampooed using a commercially available product, rinsed and towel blotted to slight dampness. Each of the tresses was then saturated with about 2-3 milliliters of waving lotion, wrapped on rollers and the waving lotion reapplied in the same amount. The tresses were maintained at room temperature and allowed to process. The treated tresses were divided into three groups.

Processing of the first group of tresses was stopped after ten minutes by rinsing with warm tap water and neutralization with a proportional amount of supplied neutralizer. Processing of the second group of tresses was stopped after twenty minutes by rinsing and neutralization as already described. These tresses were called Groups A and Group B, respectively, and were controls.

The third group of tresses, Group C, were treated by the method of this invention. These tresses were thoroughly rinsed with warm tap water after processing for ten minutes at room temperature, and then the temperature of the damp hair was raised by placing the covered tresses under a heated hair dryer for ten minutes. The hair reached a

-20-

temperature of about 48° C. These tresses were then neutralized as were the tresses of Groups A and B.

All of the tresses were rinsed with warm tap water and dried after completion of the neutralization step.

The tresses were evaluated for their curl properties after drying using a blind test with experienced workers. The Group C tresses, treated by the method of this invention were adjudged to have better initial curl as well as longer lasting curl with deeper undulations after repeated shampoo treatments than did the tresses of either of Groups A or B.

Further Comparative Ammonium
Example 2: Thioglycolate Tress Waving

Tresses (Example 1) were waved with another commercially available waving lotion. This waving lotion contained ammonium thioglycolate and diammonium dithiodiglycolate (10 percent as thioglycolic acid and 2.5 percent as dithiodiglycolic acid, respectively) and had a pH value of 8.9. Shampooing, application of waving lotion, wrapping on rollers, rinsing, neutralization, rinsing and drying were accomplished as described in Example 1 except that the tresses were covered with a plastic film during processing with the lotion. A proportional amount of the neutralizer supplied with this commercial product was used.

In this comparison, one group of tresses, Group D, was processed under a plastic film at room temperature for fifteen minutes, rinsed, neutralized, rinsed and dried. This group was the control.

The second group of tresses, Group E, was processed according to this invention. Processing at ambient, room, temperature was stopped after ten
minutes and the waving lotion was removed by
rinsing.  The rinsed, wound, partially reduced, damp
hair was re-covered with the plastic, placed under a
heated hair dryer and maintained there for a total
heating time of ten minutes.  The hair was raised to
a temperature of about 48° C.  The Group E tresses
were then neutralized with the same amount of the
supplied neutralizer, rinsed and dried.

Evaluation of these tresses was performed as
in Example 1.  This evaluation indicated that tresses
treated according to this invention had better
initial curl as well as more lasting curls then did
those of Group D; i.e., the Group E curls showed much
less curl relaxation at least through five shampoos.

Example 3:  On Head Comparison

The hair on six models' heads was waved
using the commercially available waving lotion and
its neutralizer of Example 2, using the method of
this invention for each model.

After shampooing and towel blotting to
dampness, each of the models' hair was saturated with
waving lotion, wrapped on rollers and the hair was
resaturated according to the product's directions.
Each model's hair was then covered with a plastic cap
and allowed to process at room temperature for ten
minutes, and was thereafter rinsed with running, warm
tap water to remove the waving lotion substantially
completely from the hair.  The damp hair was
re-covered with a plastic cap and the models sat
beneath salon-type heated hair dryers for ten
minutes; the models' hair temperature being raised to
about 48° C.  Following the heating step, each
model's hair was neutralized with the supplied

-22-

hydrogen peroxide neutralizer, rinsed, evaluated, set and dried.

The curl level and curl spring after rinsing of the neutralizer (Initial) and one week later after a shampoo (Return) were determined by at least two qualified evaluators. The results of these evaluations are given in the Table below.

## Table

| | | Waving Effectiveness | | | |
|---|---|---|---|---|---|
| | | Curl Level* | | Curl Spring** | |
| | Model No. | Initial | Return | Initial | Return |
| | 1 | 10 | 10 | 5 | 5 |
| | 2 | 10 | 10 | 5 | 4 |
| | 3 | 10 | 10 | 5 | 5 |
| | 4 | 10 | 10 | 5 | 5 |
| | 5 | 10 | 9 | 5 | 4 |
| | 6 | 10 | 10 | 5 | 5 |
| | Average | 10 | 9.8 | 5 | 4.7 |

*Curl level is a measure of the amount of wet curl imparted to waved hair, and is rated on a scale of 1 to 10; 10 being the best.

**Curl spring is a measure of the bounciness or spring back to its original shape and size when a wet curl is deformed. Curl spring is rated on a scale of 1 to 5, 5 being the best.

The results illustrated by the data in the above Table demonstrate the effectiveness of the instant method in that when a usual waving process is utilized, the Initial curl level is usually rated at about 9-9.5 and the Initial curl spring is usually rated at about 4.5. At the time of taking the Return data, an average relaxation of about 1 rating unit in

-23-

curl level and about 0.5-1 rating unit in curl spring are observed when the waved hair curls are compared to the day of waving results. As can be seen from the average values at the bottom of the Table, the rating values for Initial curl levels and Initial curl spring using the method of this invention are greater than those usually observed, and the method of this invention provided less than expected relaxation at the time the Return data were taken.

Example 4:
        Tress Wave Comparison With
        Glyceryl Thioglycolate Lotion

Virgin brown hair tresses (Example 1) were waved using a commercially available waving lotion which contained glyceryl thioglycolate (22 percent) and had a pH value of 6.9. These tresses were neutralized with the hydrogen peroxide neutralizer supplied with the waving lotion. The treated tresses were divided into two groups.

The first set of tresses, Group F, was wound damp on rollers after shampooing, saturated with waving lotion, covered with a plastic film and processed by heating to a temperature of about 48° C. with a heated hair dryer for a total heating time of twenty minutes. These tresses were then rinsed, neutralized, rinsed and dried. This group was a control.

The second set of tresses, Group G, was wound damp on rollers after shampooing, saturated with waving lotion, covered with a plastic film and processed by heating to a temperature of about 48° C. with a heated hair dryer for a total heating time of ten minutes. The hair was then rinsed substantially free of waving lotion with running tap water, thereby cooling the wrapped, partially reduced hair. The damp, rinsed hair was then placed under the plastic film again, and again heated as before for a second ten minute period. These tresses were then neutralized, rinsed and dried.

Evaluation of the waved tresses, both when wet and dry, indicated that both procedures produced good curls. However, the curls of Group G, produced by the method of this invention, were judged to have deeper undulated and more natural looking curls than were those of Group F.

Comparative Waving Using
Example 5:  Heated Clamps

Virgin brown tresses (Example 1) were treated with the waving lotion of Example 1 and neutralized with a proportional amount of the hydrogen peroxide solution supplied therewith.

The tresses were allowed to process in straight configuration for five minutes after saturation with waving lotion at ambient, room temperature. The waving lotion was then rinsed from all of the tresses, and the rinsed tresses were wound on curlers. The treated tresses were divided into two groups.

The damp, rinsed tresses of Group H were processed for an additonal six minutes at room temperature, neutralized, rinsed and dried. This group was the control.

The damp, rinsed tresses of Group I were processed for six minutes using a commercially available hair clamp which was previously heated to about 105° C. to cover each wrapped tress. The hair temperature was not measured during this heating period, but similar, previous, determinations have indicated that the hair temperature would be raised to about 50° C. during the latter, six minute, processing. The Group H tresses were thereafter neutralized, rinsed and dried.

Comparison of the tresses of Group H with those of Group I indicated that the Group I tresses, treated in accordance with this invention, had a good curl while those of Group H had no curl. This comparison thus illustrates the importance of heating the partially reduced hair during the keratin disulfide-thiol reorientation (creep) period.

On Head Waving Using
Example 6: Heated Clamps Or Hair Dryer

The hair of fifteen models with normal hair was shampooed and towel blotted. Thereafter, the models' hair was partially reduced with a thickened waving lotion containing monoethanolammonium thioglycolate (7.4 weight percent as thioglycolic acid) and a sufficient further amount of monoethanolamine to raise the pH value to 9.4.

The lotion was applied to the models' hair and maintained in contact therewith for 10 minutes. The waving lotion was thereafter thoroughly rinsed from the hair, and the resulting partially reduced models' hair was wound on permanent waving rods.

The hair of nine of the models was then heated using preheated clamps, as described in Example 5, the clamps being maintained on the hair for six minutes. The hair of the remaining six models was covered with a plastic cap and heated under a pre-heated, salon-type hair dryer for ten minutes, as described in Example 3. The hair of all fifteen models was then neutralized with a neutralizer which contained 2.2 percent hydrogen peroxide.

Both heating procedures produced good waves and left the waved hair having good condition.

Hair Condition Study Example 7: After Waving Tresses

The condition of hair waved according to the present invention was compared to that of hair waved using a leading, salon-type waving product. The "liquid retention" method discussed by Wolfram and Underwood in Textile Research Journal, 36(11), pp. 947-953 (1966)) was utilized for the comparison, with distilled water replacing the buffer used in above article, a 15 minute soaking time and centrifugation for 30 minutes. Liquid retention determinations reflect the condition of the hair measured by relative porosity of the fibers.

Brown hair tresses (Example 1) were used. One set of tresses was waved using the lotion, heated clamps, and waving and neutralizing procedures described in Example 6. Package directions were followed for waving and neutralizing with the leading salon-type product. The waving lotion of that product, after mixing with the supplied hydrogen peroxide to cause chemical heating, had a pH value of 9.0 and contained a thiocarboxylate reducing agent (about 9.5 weight percent as thioglycolic acid) as well as a dithocarboxylic acid (about 5 percent by weight as dithiodiglycolic acid).

The waved hair from both treatments was shampooed after neutralization, and both treatments were found to impart similar wave levels. The procedure of this invention gave a slightly better wave. Liquid retention data for hair fibers cut from (a) control tresses which were not waved, (b) tresses waved according to this invention and (c) tresses

-27-

waved using the leading salon-type product are set out in the Table below.

Table

Liquid Retention Data

(Percent Liquid Retained Based

Upon Dry Hair Weight)

| Control | This Invention | Leading Product |
|---------|----------------|-----------------|
| 36.8 | 45.7 | 55.1 |

The above data illustrate the improved hair condition obtained using the instant invention as compared to the process employed with the leading product. The improved condition of the hair coupled with the comparable level of waving amply demonstrates a benefit of the instant invention.

On Head Bisulfite
Straightening Using
Example 8:  Pre-heated Rollers and Clamps

The hair of fifty-four models having naturally curly hair was treated with a waving lotion containing ammonium bisulfite to partially straighten or relax the models' hair. Two different thickened waving lotions in two different processing conditions were utilized. The first bisulfite lotion contained 1.0 molar ammonium bisulfite (9.9 weight percent) while the second bisulfite lotion contained 1.25 molar ammonium bisulfite (12.4 weight percent). The pH value of each of the lotions was about 6.7.

In the first method, to convert naturally curly hair to a more relaxed configuration, dry hair was first treated with the first (above) lotion, the

-28-

treated hair was covered with a plastic cap, and then processed under a heated salon-type hair dryer (Example 3) for from 15 to 20 minutes to form partially reduced hair which was then combed through. The partially reduced hair was then shampooed with a commercially available, alkaline shampoo having a pH value of 9 to substantially remove the bisulfite ion from the hair, and partially reverse the disulfide bond cleavage process. The shampoo was thereafter removed from the hair using tap water.

The damp, shampooed hair was then wrapped on pre-heated rollers with the diameter of the rollers being selected on the basis of the amount of curl desired. The hair was left in contact with the heated roller for six minutes, raising the models' hair to an average temperature of about 43° C. The hair was thereafter treated with the neutralizer (hydrogen peroxide, 2.2 percent by weight) for a five minute time period. The neutralizer was rinsed out of the hair and the hair styled.

A more curly configuration than obtained above was achieved with the second method. In this method, the hair was treated as above using the second lotion, but instead of using heated rollers after the shampooing, conventional waving rods, having relatively smaller diameters, were used in conjunction with pre-heated clamps which were placed over the waving rods to heat the hair. In this treatment, the average temperature of the models' hair was raised to about 50° C.

All of the models were satisfied on the day of treatment, and fifty-two of the fifty-four models were satisfied one to ten weeks after treatment. In response to questioning, the models were particularly

-29-

pleased with the degree of relaxation and the new curl configuration imparted to the hair. They also responded that their hair was easier to style and maintain after the treatment.

On Head Bisulfite Waving

Example 9: Using A Salon-Type Hair Dryer

Models' naturally curly hair was treated with the second bisulfite lotion of Example 8 with the hair being partially reduced in accordance with the second method of that Example. After treatment with the alkaline shampoo, the hair was wrapped on conventional waving rods and covered with a plastic cap. The hair was then heated for ten minutes using a pre-heated, salon-type hair dryer as discussed in Example 3. Neutralization was effected as discussed in Example 8. Good, new curl configurations were imparted to the models' hair waved in this Example.

The present invention has been described with respect to preferred embodiments. It will be clear to those skilled in the art that modifications and/or variations of the disclosed methods can be made without departing from the scope of the invention set forth herein. The invention is defined by the claims which follow.

-30-

CLAIMS:

1. A method of reductively waving or straightening hair comprising the steps of:

applying a first composition containing a reducing agent capable of reductively breaking the disulfide bonds in hair keratin to contact hair fibers;

maintaining said contact for a period of time sufficient to form partially reduced hair;

rinsing said first composition substantially completely from said partially reduced hair;

raising the temperature of the rinsed hair while in partially reduced condition to from about 35° C. to about 100° C., and maintaining said hair at said raised temperature for a period of about 1 to about 60 minutes, said hair being under a tensile force which is applied at any time prior to raising the temperature of said hair; and

thereafter applying a neutralizing composition to the heated hair to rebuild the broken hair disulfide bonds.

2. The method according to claim 1 wherein said first composition contains said reducing agent in an amount of from about 0.5 to about 30 percent by weight.

3. The method according to claim 1 wherein said first composition is maintained in contact with said hair for a period of from about 2 to about 30 minutes.

4. The method according to claim 1 wherein said rinsed, partially reduced hair, after being raised to a temperature of from about 35° C. to about 100° C., is maintained at said temperature for a total period of from about 2 to about 30 minutes.

5. The method according to claim 1 wherein said tensile force is applied externally to the hair.

6. The method of claim 5 wherein said external tensile force is applied prior to the application of said first composition to said hair fibers.

7. The method of claim 5 wherein said external tensile force is applied after the application of said first composition to said hair fibers and prior to said rinsing.

8. The method of claim 5 wherein said external tensile force is applied after said rinsing.

9. The method according to claim 5 wherein said external tensile force is provided by wrapping the hair on rollers.

10. The method according to claim 1 wherein said first composition is maintained in contact with said hair until at least about 10 percent of said disulfide bonds in said hair keratin are broken.

11. A method of reductively waving or straightening hair comprising the steps of:

applying a first composition containing about 0.5 to about 30 percent by weight of agent capable of reductively breaking the disulfide bonds in hair keratin to contact hair fibers;

maintaining said contact for a period of time of from about 2 to about 30 minutes to form partially reduced hair;

rinsing said first composition substantially completely from said partially reduced hair;

raising the temperature of the rinsed hair while in partially reduced condition to from about 40° C. to about 55° C., and maintaining said hair at said temperature for a period of about 2 to about 30 minutes, said hair being wrapped on rollers at any time prior to raising the temperature of said hair; and

-32-

thereafter applying a neutralizing composition to the heated hair to rebuild the broken hair disulfide bonds.

12. The method according to claim 11 wherein said hair is wrapped on rollers prior to the application of said first composition.

13. The method according to claim 11 wherein said hair is wrapped on rollers after applying said first composition.

14. The method according to claim 11 wherein said rinsed, partially reduced hair is wrapped on rollers.

15. The method according to claim 11 wherein said first composition includes a water-soluble thiol-containing reducing agent selected from the group consisting of a mercaptocarboxylic acid and the alkali metal or ammonium salt of a mercaptocarboxylic acid present at about 1 to about 20 weight percent of said composition.

16. The method according to claim 15 wherein said neutralizing composition includes an oxidizing agent capable of rebuilding said broken hair disulfide bonds.

17. The method according to claim 11 wherein said first composition includes a bisulfite ion reducing agent present at about 5 to about 15 weight percent of said composition.

18. The method of claim 17 wherein a portion of the disulfide bonds reduced by said bisulfite ion reducing agent are rebuilt prior to said heating step.

19. A method of reductively waving or straightening hair comprising the steps of:

applying a first composition containing about 1 to about 20 weight percent of a water-soluble thiol-containing reducing agent capable of reductively breaking the disulfide bonds in hair keratin to contact hair fibers, said reducing agent being selected from the group consisting of a mercaptocarboxylic acid and the alkali metal or ammonium salts of a mercaptocarboxylic acid;

maintaining said contact with said hair fibers for a period of from about 2 to about 20 minutes to form partially reduced hair;

rinsing said first composition substantially completely from said partially reduced hair;

raising the temperature of the rinsed, partially reduced, hair to from about 40° C. to about 55° C. and maintaining said hair at said temperature for a total of from about 2 to about 30 minutes, said hair being wrapped on rollers at any time prior to raising the temperature of said hair; and

applying a second composition to the heated hair, said second composition containing an oxidizing agent capable of rebuilding said broken disulfide bonds.

20. A method of reductively waving or straightening hair comprising the steps of:

applying a first composition containing about 5 to about 15 weight percent of a bisulfite ion reducing agent capable of reductively breaking the disulfide bonds in hair keratin to contact said hair;

maintaining said contact for a period of from about 2 to about 20 minutes to form partially reduced hair;

rinsing said first composition substantially completely from said partially reduced hair;

wrapping said rinsed, partially reduced hair on rollers;

-34-

raising the temperature of the wrapped, rinsed partially reduced hair to from about 40°C. to about 55°C. and maintaining said hair at said temperature for a total of from about 2 to about 30 minutes; and

applying a second composition to the heated hair, said second composition containing an oxidizing agent capable of rebuilding said broken disulfide bonds.

21. The method according to claim 20 including the additional step of rebuilding a portion of the broken disulfide bonds prior to said heating step.

22. The method according to claim 21 wherein said portion of the broken disulfide bonds are rebuilt by rinsing said first composition substantially from said partially reduced hair with a shampoo composition having a pH value of from about 8.5 to about 10.5.